(19) Europäisches Patentamt
European Patent Office
Office européen des brevets

(11) EP 3 305 760 A1

(12) EUROPEAN PATENT APPLICATION
published in accordance with Art. 153(4) EPC

(43) Date of publication:
11.04.2018 Bulletin 2018/15

(21) Application number: 16803316.5

(22) Date of filing: 31.05.2016

(51) Int Cl.:
*C07C 303/22* (2006.01) *C07C 309/46* (2006.01)

(86) International application number:
PCT/JP2016/065945

(87) International publication number:
WO 2016/194881 (08.12.2016 Gazette 2016/49)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 01.06.2015 JP 2015111304

(71) Applicant: AJINOMOTO CO., INC.
Chuo-ku
Tokyo 104-8315 (JP)

(72) Inventors:
- OKADO Kotaro
Yokkaichi-shi
Mie 510-0885 (JP)
- ABE Nobuteru
Kawasaki-shi
Kanagawa 210-8681 (JP)
- MURONOI Shin
Yokkaichi-shi
Mie 510-0885 (JP)
- KOBAYASHI Yasuhisa
Yokkaichi-shi
Mie 510-0885 (JP)
- NIWA Seiji
Yokkaichi-shi
Mie 510-0885 (JP)
- MATSUZAWA Toshihiro
Kawasaki-shi
Kanagawa 210-8681 (JP)

(74) Representative: Dunne, Paul David
Mewburn Ellis LLP
City Tower
40 Basinghall Street
London EC2V 5DE (GB)

# (54) METHOD FOR PRODUCING ALKYLAMINE DERIVATIVE AND PRODUCTION INTERMEDIATE OF ALKYLAMINE DERIVATIVE

(57) A method for producing an alkylamine derivative having a urea bond represented by formula (I), or a salt thereof, comprises the following steps (a) and (b),
step (a):

EP 3 305 760 A1

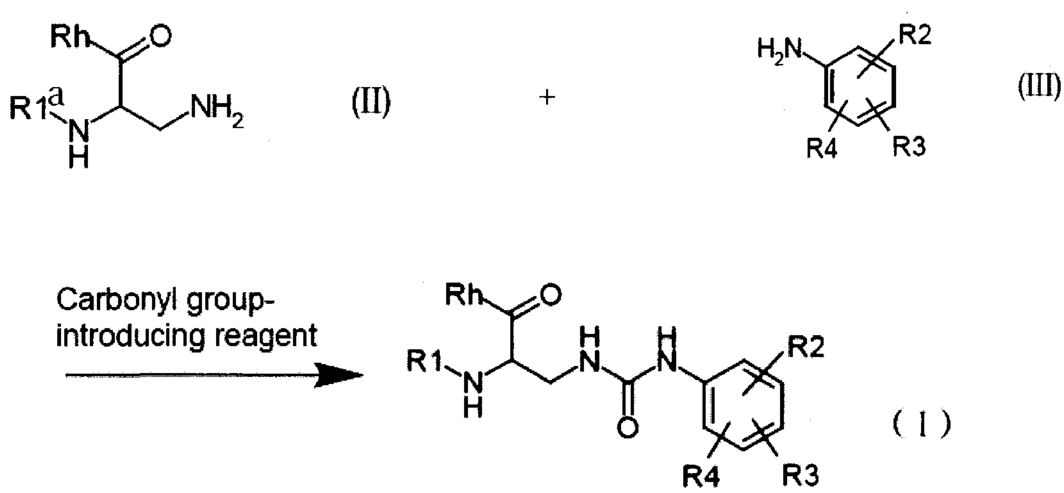


(II) + (III)

(I)

and

step (b): deprotecting as necessary the reaction product obtained in step (a). The production method suitable for industriallization of the alkylamine derivative having a urea bond represented by formula (I), which is a compound highly useful as an agent having CaSR agonist effects is provided.

## Description

TECHNICAL FIELD

The present invention relates to a novel method for producing an alkylamine derivative and its novel production intermediate.

BACKGROUND ART

In recent years, by progression of research on diseases that are improved by the activation of CaSR (calcium-sensing receptor), such as diarrhea, peptic ulcer, hyperparathyroidism, and secondary hyperparathyroidism under maintenance dialysis, compounds having an effect of activating CaSR are expected for being applied to therapeutic agents or prophylactic agents. For example, it has been revealed that the CaSR agonist, Cinacalcet (CCT) has an effect of suppressing the secretion of parathyroid hormone by acting on CaSR in the parathyroid gland to increase the $Ca^{2+}$ sensitivity of CaSR (Non Patent Literature 1), and is commercially available as a therapeutic drug against secondary hyperparathyroidism of dialysis patients (Non Patent Literature 2).

The applicant of the present invention has already filed patent applications for the inventions relating to alkylamine derivatives having CaSR agonist effects and being highly useful as therapeutic agents or prophylactic agents against diseases that are improved by the activation of CaSR (Patent Literature 1 and Patent Literature 2).

Patent Literature 1 and Patent Literature 2 disclose a method shown in the following scheme as a general method for producing an alkylamine derivative having a urea bond included in general formula (I-c) (see Patent Literature 1 for symbols in the formula).

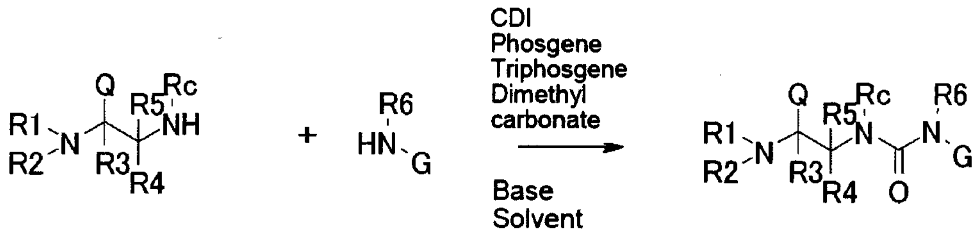


(2c) (3) (I-c)

A urea derivative (I-c) can be produced by dissolving or suspending amine or a salt thereof of an alkylamine derivative represented by general formula (2c) and an amine derivative represented by general formula (3) in an appropriate solvent, mixing with a condensation agent such as CDI (carbonyldiimidazole), phosgene, and triphosgene, or a carbonyl source such as dimethyl carbonate in the presence or the absence of a base such as triethylamine and pyridine, and when necessary, followed by cooling, heating or the like of the reaction system.

However, processes disclosed in the examples of Patent Literature 1 or Patent Literature 2 are not suitable as industrial processes in terms of yield. Moreover, Patent Literature 1 discloses the production method that requires purification using reverse-phase high-performance liquid chromatography. Therefore, whereas an alkylamine derivative can be expected as a therapeutic agent or a prophylactic agent useful against diseases that are improved by the activation of CaSR, a novel method capable of producing the alkylamine derivative in an industrially more efficient manner than that in conventional production methods has been desired.

Patent Literature 1: International Publication No. WO2011/108690
Patent Literature 2: Japanese Patent Laid-Open No. 2013-63971

Non Patent Literature 1: Current Opinion Pharmacology (2002), 2: 734-739
Non Patent Literature 2: "REGPARA tablets (R)25mg/REGPARA tablets (R) 75 mg" documents attached to medical drugs, Revised January 2010 <5th Edition>

DISCLOSURE OF THE INVENTION

The present invention is intended to provide an industrially suitable method for producing an alkylamine derivative having a urea bond represented by formula (I), which is a compound highly useful as an agent having CaSR agonist effects. The present invention particularly relates to a method for conveniently producing the target alkylamine derivative having a urea bond with high quality in high yields without requiring purification by reverse-phase high-performance liquid chromatography.

As a result of intensive studies to achieve the above intention, the present inventors have discovered an industrial manufacturing method for producing the following alkylamine derivative having a urea bond, and a novel intermediate to be used in the process, and thus have completed the present invention.

Hence, the present invention provides an industrial manufacturing method for producing an alkylamine derivative having a urea bond represented by the following formula (I), and, an intermediate useful in the production thereof.

Specifically, the present invention relates to the following [1] to [15].

[1] A method for producing a compound represented by formula (I) or a salt thereof:

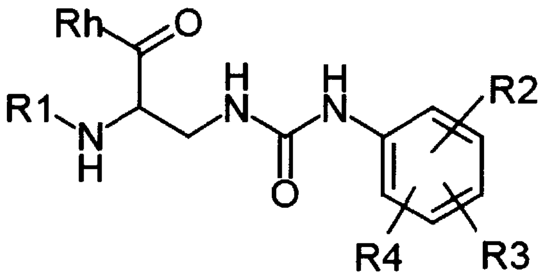

( I )

wherein R1 represents a hydrogen atom,
Rh represents a hydroxy group, a $C_{1-6}$ alkoxy group, or a benzyloxy group,
R2 represents a sulfo group,
R3 and R4 each independently represent a hydrogen atom, a substituted or unsubstituted $C_{1-6}$ alkyl group, a halogen atom, a hydroxy group, a $C_{1-6}$ alkoxy group, a nitro group, or an amino group,
comprising the following steps (a) and (b):

(a) dissolving or suspending a compound represented by formula (II) or a salt thereof:

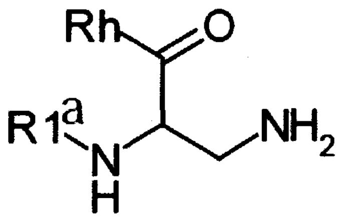

(II)

wherein $R1^a$ represents a hydrogen atom, or a protecting group for an amino group, a carbonyl group-introducing reagent, and a compound represented by formula (III) or a salt thereof:

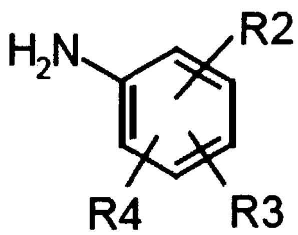

(III)

in a solvent, for reaction in the presence or the absence of a base; and
(b) deprotecting as necesssary the reaction product obtained in step (a).

[2] The production method according to [1] above, wherein in formula (II), $R1^a$ represents a benzyloxycarbonyl group or t-butoxycarbonyl group.

[3] The production method according to [1] or [2] above, wherein in formula (III), R3 and R4 each independently

represent a hydrogen atom, an unsubstituted $C_{1-6}$ alkyl group, a halogen atom, or a hydroxy group.

[4] The production method according to any one of [1] to [3] above, wherein the carbonyl group-introducing reagent is a chloroformic ester, carbonyldiimidazole, phosgene, triphosgene, or dimethyl carbonate.

[5] The production method according to any one of [1] to [3] above, wherein in step (a), the carbonyl group-introducing reagent is carbonyldiimidazole, the base is absent, and the solvent is one or two or more solvents selected from acetone, methyl ethyl ketone, methyl isobutyl ketone, dichloromethane, tetrahydrofuran and acetonitrile.

[6] The production method according to any one of [1] to [3] above, wherein in step (a), the carbonyl group-introducing reagent is a chloroformic ester, the base is one or two or more bases selected from triethylamine, pyridine, and diisopropylethylamine, and the solvent is one or two or more solvents selected from acetonitrile, propionitrile, dichloromethane, acetone, N,N-dimethylformamide and tetrahydrofuran.

[7] The production method according to [6] above, wherein the chloroformic ester is methyl chloroformate, ethyl chloroformate, phenyl chloroformate, 4-chlorophenyl chloroformate or 4-nitrophenyl chloroformate.

[8] A method for producing a compound represented by formula (I), or a salt thereof:

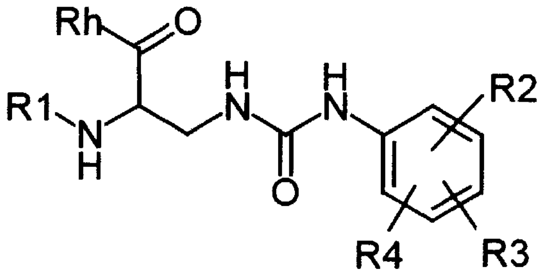

( I )

wherein R1 represents a hydrogen atom,
Rh represents a hydroxy group, a $C_{1-6}$ alkoxy group, or a benzyloxy group,
R2 represents a sulfo group,
R3 and R4 each independently represent a hydrogen atom, a substituted or unsubstituted $C_{1-6}$ alkyl group, a halogen atom, a hydroxy group, a $C_{1-6}$ alkoxy group, a nitro group, or an amino group,
comprising the following steps (a-1), (a-2) and (b):

(a-1) dissolving or suspending a compound represented by formula (III) or a salt thereof:

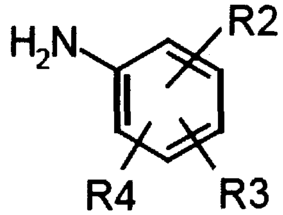

(III)

in a solvent with a chloroformic ester, for reaction in the presence or the absence of a base, to obtain a reaction product containing a compound of formula (IVb) or a salt thereof,:

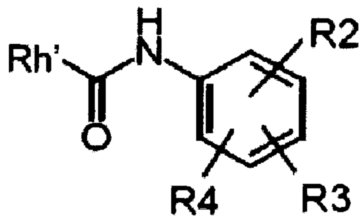

(IVb)

wherein Rh' represents a $C_{1-6}$ alkoxy group, a benzyloxy group, or a phenoxy group,

(a-2) reacting the reaction product obtained in the above step (a-1) containing the compound of formula (IVb) or a salt thereof, with a compound represented by formula (II) or a salt thereof:

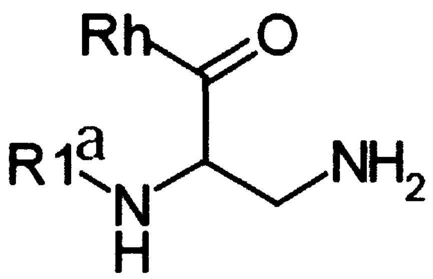

(II)

wherein R1ᵃ represents a hydrogen atom, or a protecting group for an amino group, and
(b) deprotecting as necessary the reaction product obtained in step (a-2).

[9] The production method according to any one of [1] to [8] above, wherein R1ᵃ represents a t-butoxycarbonyl group, and in the above step (b), the deprotecting reagent which is hydrochloric acid, sulfuric acid, p-toluenesulfonic acid, methanesulfonic acid, or trifluoroacetic acid is used.
[10] The production method according to any one of [1] to [8] above, wherein R1ᵃ represents a benzyloxycarbonyl group, and in the above step (b), the deprotecting reagent which is hydrogen bromide/acetic acid is used, or a hydrogenation reaction is performed using palladium carbon.
[11] The production method according to any one of [1] to [8] above, wherein Rh represents a t-butoxy group, and in the above step (b), the deprotecting reagent which is hydrochloric acid, formic acid, p-toluenesulfonic acid, trifluoroacetic acid, or potassium hydroxide is used.
[12] The production method according to any one of [1] to [8] above, wherein Rh represents a methoxy group, and in the above step (b), the deprotecting reagent which is sodium hydroxide, potassium hydroxide, or lithium hydroxide is used.
[13] A compound represented by the following formula or a salt thereof:

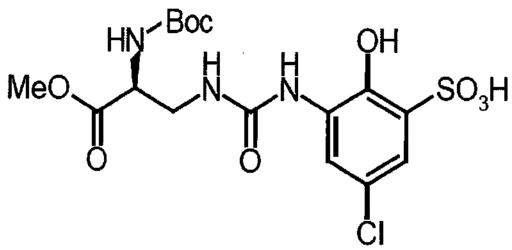

,

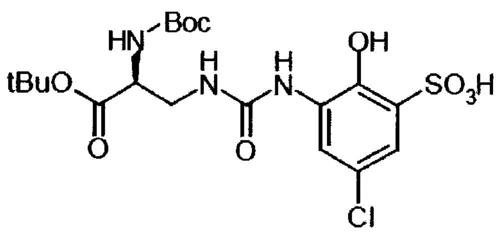

,

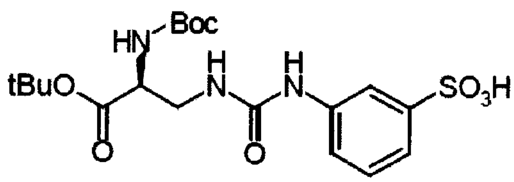

,

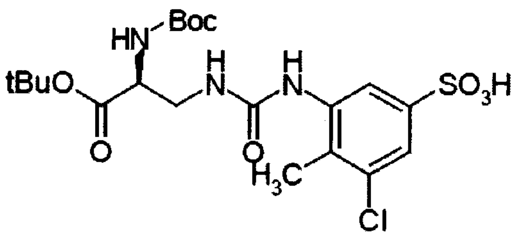

,

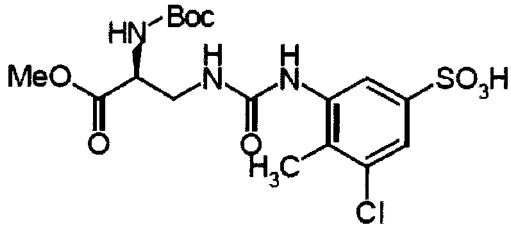

or

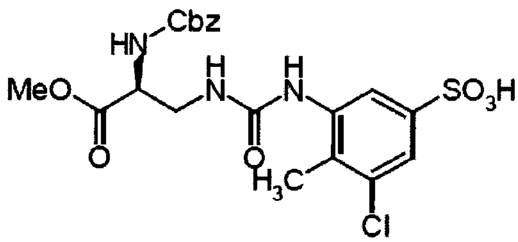

[14] A compound represented by the following formula or a salt thereof:

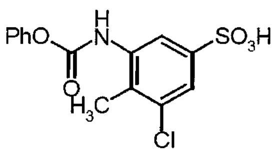

,

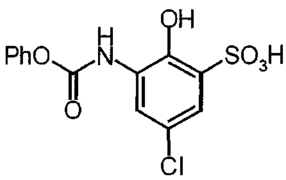

,

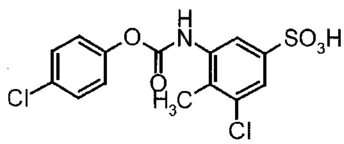

or

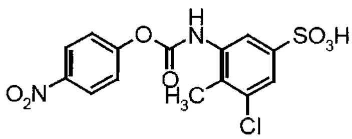

[15] The production method according to any one of [1] to [12] above, which does not require purification by reverse-phase high-performance liquid chromatography.

The present invention provides a production method appropriate for mass synthesis of an alkylamine derivative and a novel intermediate. With the production method of the present invention, the target compound of formula (I), an alkylamine derivative having a urea structure, can be produced with high purity in high yields without requiring purification by reverse-phase high-performance liquid chromatography, through the use of a chloroformic ester, CDI, phosgene, or triphosgene as a carbonyl group-introducing reagent and if necessary a predetermined deprotection step.

Description of Embodiments

"$C_{1-6}$ alkyl group" is a monovalent group induced by removing any one hydrogen atom from linear- and branched-chain aliphatic hydrocarbons having 1-6 carbons. Specific examples thereof include a methyl group, an ethyl group, a n-propyl group, an isopropyl group, a n-butyl group, an isobutyl group, a sec-butyl group, a t-butyl group, a pentyl group, an isopentyl group, a 2-methylbutyl group, and a hexyl group. Preferably, it is a $C_{1-3}$ alkyl group.
"$C_{1-6}$ alkoxy group" refers to $C_{1-6}$ alkyl-O-. Specific examples thereof include a methoxy group, an ethoxy group, a 1-propoxy group, a 2-propoxy group, a n-butoxy group, an isobutoxy group, a sec-butoxy group, a t-butoxy group, a 1-pentyloxy group, a 2-pentyloxy group, a 3-pentyloxy group, a 2-methyl-1-butyloxy group, a 3-methyl-1-butyloxy group, a 2-methyl-2-butyloxy group, a 3- methyl-2-butyloxy group, a 2,2-dimethyl-1-propyloxy group, a 1-hexyloxy group, a 2-hexyloxy group, and a 3-hexyloxy group. Preferably, it is a $C_{1-3}$ alkoxy group.
"Halogen atom" refers to a fluorine, chlorine, bromine, or iodine atom, for example.
As a "protecting group for an amino group" represented by $R1^a$, any protecting group can be used as long as it does not inhibit the reaction. Examples thereof that can be preferably used herein include carbamate-based protecting groups (e.g., a benzyloxycarbonyl group, a t-butoxycarbonyl group, and a 9-fluorenylmethyloxycarbonyl group), amide-based protecting groups (e.g., a formyl group, an acetyl group, and a trifluoroacetyl group), aminoacetal-based protecting groups (e.g., a benzyloxymethyl group), benzyl-based protecting groups (e.g., a benzyl group), and in addition to these examples, a trityl group. Of these examples, a benzyloxycarbonyl group, and a t-butoxycarbonyl group are particularly preferable.
The present invention relates to a method for producing a compound represented by the following formula (I) or a salt thereof, and preferably an industrial manufacturing method.
Here, "industrial manufacturing method" refers to an efficient method for industrially producing a target product, and is a convenient method for producing a target product in high yields and/or with high purity. Specifically, the industrial manufacturing method is a production method requiring no purification step inappropriate for the industrial manufacturing method, such as purification by reverse-phase high-performance liquid chromatography.
Each step of the production method of the present invention is explained in detail as follows.

Step (a)

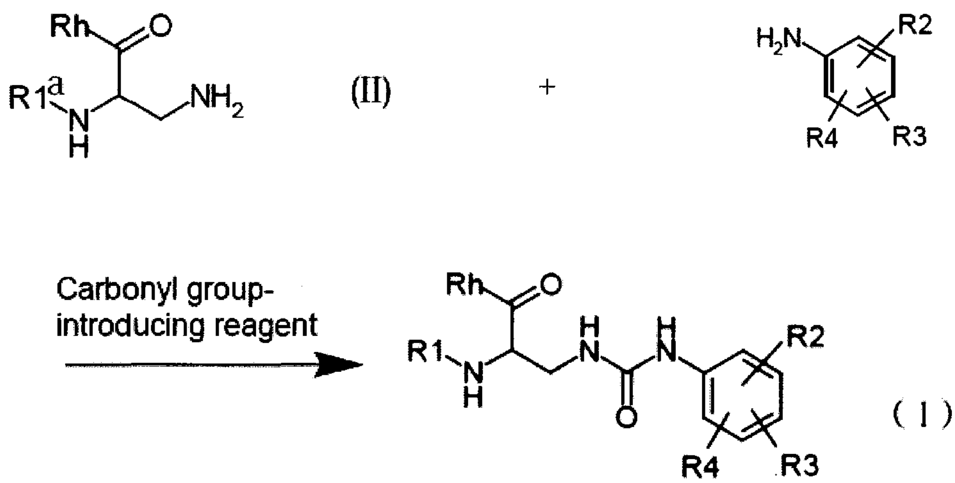


(II) + (III)

(I)

(Symbols in this formula are as defined above.)

This step is a step of obtaining the compound of formula (I) or a salt thereof by reacting a compound of formula (II) or a salt thereof with a compound of formula (III) or a salt thereof using a carbonyl group-introducing reagent.
The above reaction is generally performed in a solvent and the solvent may be any solvent as long as it does not inhibit the reaction. Examples thereof include hydrocarbons (e.g., n-hexane, n-heptane, petroleum ether, benzene, toluene, and xylene), halogenated hydrocarbons (e.g., dichloromethane, chloroform, carbon tetrachloride, dichloroethane, and chlorobenzene, etc.), ethers (e.g., diethyl ether, diisopropyl ether, dioxane, tetrahydrofuran, dimethoxy ethane, and ethylene glycol dimethyl ether, etc.), amides (e.g., N,N-dimethylformamide, N,N-dimethylacetamide, and N-methyl-2-pyrrolidone, etc.), esters (e.g., ethyl acetate, isopropyl acetate, butyl acetate, propyl acetate, and methyl

acetate, etc.), nitriles (e.g., acetonitrile, and propionitrile), sulfoxides (e.g., dimethyl sulfoxide, and sulfolane, etc.), ketones (e.g., acetone, methyl ethyl ketone, methyl isobutyl ketone, and cyclohexanone, etc.), acids (e.g., formic acid, acetic acid, propionic acid, trifluoroacetic acid, and sulfuric acid, etc.), water, and alcohols (e.g., methanol, ethanol, and propanol, etc.). These solvents can be used independently or as a mixed system of two or more thereof.

The above reaction is performed in the presence or the absence of a base, and examples of the base include 1) inorganic bases, such as hydroxides of alkali metal or alkaline-earth metal (e.g., sodium hydroxide, potassium hydroxide, lithium hydroxide, and barium hydroxide, etc.), carbonates of alkali metal or alkaline-earth metal (e.g., sodium carbonate, potassium carbonate, and cesium carbonate, etc.), and hydrogen carbonates of alkali metal or alkaline-earth metal (e.g., sodium hydrogen carbonate, and potassium hydrogen carbonate, etc.), and 2) organic bases, such as basic heterocyclic compounds such as amines or pyridine, e.g., triethylamine, diisopropylethylamine, N-methylmorpholine, dimethylaminopyridine, DBU (1,8-diazabicyclo[5.4.0]-7-undecene), and DBN (1,5-diazabicyclo[4.3.0]non-5-ene), imidazole, and 2,6-lutidine, etc. Preferably the reaction is performed in the absence of a base.

Examples of a carbonyl group-introducing reagent to be used in the method of the present invention include a chloroformic ester, carbonyldiimidazole (CDI), phosgene, triphosgene, and dimethyl carbonate, etc. An ester portion of the chloroformic ester is lower alkyl, or phenyl that may be substituted. Examples of a substituent of phenyl that may be substituted include a halogen atom and a nitro group, a methyl group, and a methoxy group. A carbonyl group-introducing reagent is preferably methyl chloroformate, ethyl chloroformate, phenyl chloroformate that may be substituted, or CDI, and is particularly preferably CDI, or phenyl chloroformate.

The order of introducing raw materials and reagents is not particularly limited. Depending on the properties of a carbonyl group-introducing reagent to be used and the compound of formula (II) or formula (III), a reaction can be performed by a method that involves adding the compound of formula (II) to a solution of a carbonyl group-introducing reagent to react followed by reacting the reaction solution with the compound of formula (III), or, a method that involves adding the compound of formula (III) to a solution of a carbonyl group-introducing reagent to react followed by reacting the reaction solution with the compound of formula (II). Furthermore, after reaction of a carbonyl group-introducing reagent with the compound of formula (II) or formula (III), the reaction compound may be isolated and then used for the next reaction, or the same may be used for the next reaction without isolation thereof.

When a carbonyl group-introducing reagent is carbonyldiimidazole (CDI), to a solution or suspension of CDI, it is preferable to add the compound of formula (III) after the introduction of the compound of formula (II) in view of reactivity and impurity reduction.

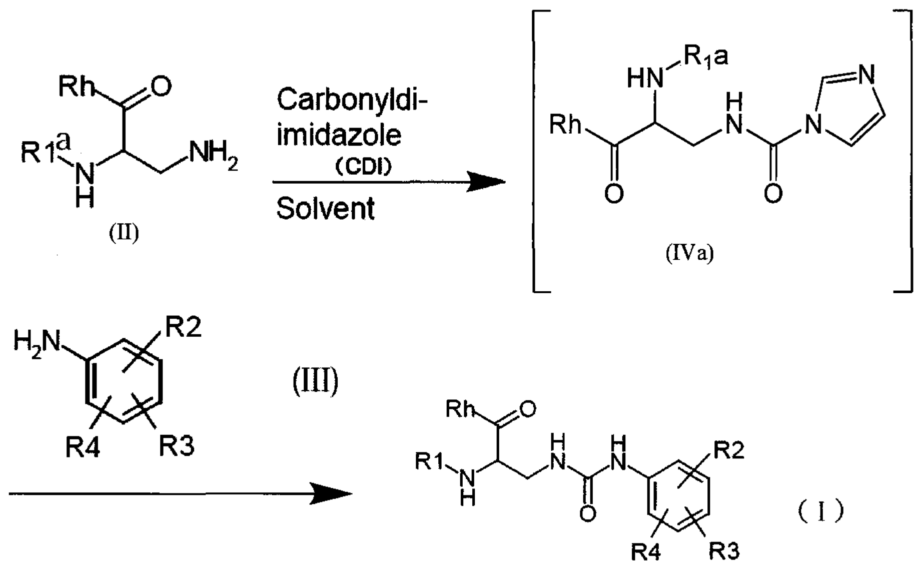

(II)
(IVa)
(III)
( I )

(Symbols in this formula are as defined above.)

A compound (IVa) may be isolated from a reaction product obtained by a reaction of the compound of formula (II) or a salt thereof with carbonyldiimidazole and then subjected to the next reaction. Preferably, the reaction product is used directly without isolation thereof and then the compound of formula (III) or a salt thereof is added.

Solvents are preferably ketones, ethers, and halogenated hydrocarbons, and are preferably acetone, acetonitrile, tetrahydrofuran, and dichloromethane. In particular, acetone is more preferable. The amount of a reaction solvent to be used herein is not particularly limited and preferably ranges from about 1 ml to about 10 ml, with respect to 1 g of the compound of formula (II). When a base is used, an organic base is preferable and pyridine is more preferable.

Preferably, the reaction is performed in the absence of a base. The amount of CDI to be used herein generally ranges from about 1 mol to about 1.5 mol, and is preferably about 1.1 mol, with respect to 1 mol of the compound of formula (II). The reaction temperature ranges from 0°C to 60°C, and preferably ranges from 5°C to 40°C. The reaction time generally ranges from about 12 hours to 24 hours.

When a carbonyl group-introducing reagent is phenyl chloroformate that may be substituted, it is preferable to add a base and add dropwise a solution of phenyl chloroformate that may be substituted, to a solution or suspension containing the compound of formula (III) in view of improvement in yield, or impurity reduction.

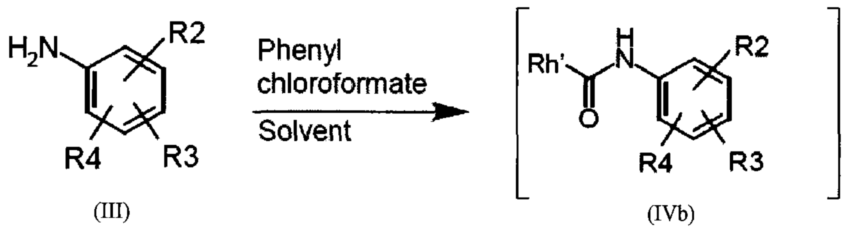


(III) (IVb)

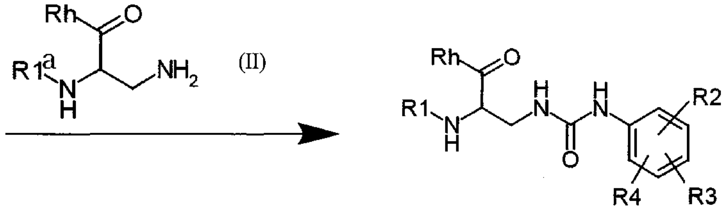

(II)

( I )

(Symbols in this formula are as defined above.)

The compound (IVb) may be isolated from a reaction product obtained by a reaction of the compound of formula (III) or a salt thereof with phenyl chloroformate that may be substituted, and then subjected to the next reaction. Preferably, the reaction product is used directly without isolation thereof, and then the compound of formula (II) or a salt thereof is added. Solvents are preferably nitriles, amides, and ethers, and are more preferably acetonitrile, N,N-dimethylformamide, and tetrahydrofuran. In particular, acetonitrile is more preferable. The amount of a reaction solvent to be used is not particularly limited, and preferably ranges from about 3 ml to about 20 ml with respect to 1 g of the compound of formula (III). A base is preferably an organic base, pyridine is more preferable in carbamate formation and triethylamine, and diisopropylethylamine are more preferable in urea formation. The amount of phenyl chloroformate to be used herein, which may be substituted, generally ranges from about 1 mol to about 1.5 mol, and is preferably about 1.1 mol with respect to 1 mol of the compound of formula (III). The amount of a base to be used is generally an amount required for activation of the reaction and preferably ranges from about 2 mol to about 6 mol with respect to 1 mol of the compound of formula (III). The reaction temperature ranges from 0°C to 70°C, and preferably ranges from 25°C to 60°C. The reaction time generally ranges from about 4 hours to 12 hours.

Step (b) (deprotection)

This step is a step that is conducted as necessary depending on a compound to be obtained. A deprotection method can be performed using a deprotecting reagent under acidic or basic conditions, or in the presence of a metal catalyst, a reductive deprotection reaction (hydrogenolysis) can be performed.

Examples of a deprotecting reagent under acidic conditions include acids such as hydrochloric acid, hydrogen bromide/acetic acid, sulfuric acid, formic acid, p-toluenesulfonic acid, methanesulfonic acid, trifluoroacetic acid, and a method for generating acids in a system of acetyl chloride/methanol or the like can be used. Of these examples, when R1 represents a tert-butoxycarbonyl group, hydrochloric acid and methanesulfonic acid are preferable, when R1 represents a benzyloxycarbonyl group, hydrogen bromide/acetic acid is preferable, and when Rh represents a tert-butoxy group, hydrochloric acid is preferable.

Examples of a deprotecting reagent under basic conditions include the above inorganic bases and organic bases, etc. Of these examples, when Rh represents a methoxy group, sodium hydroxide is preferable.

Examples of a metal catalyst to be used as a deprotecting reagent include palladium catalysts (e.g., palladium carbon, palladium hydroxide carbon, and palladium oxide), platinum catalysts (e.g., platinum carbon, and platinum oxide), rhodium catalysts (e.g., rhodium carbon), and ruthenium catalysts (e.g., ruthenium carbon). Palladium catalysts are

preferable and palladium carbon is more preferable.

In the reaction, a solvent of step (a) may be used directly, an additional solvent may be used, a solvent may be concentrated and then another solvent may be added and used, or after obtaining a proteted form another solvent may also be added. Any of the above solvents may be used as long as it does not inhibit the reaction, and can be used independently or two or more of these solvents can be used as a mixed system. As the above solvents, ketones, esters, nitriles, acids, and water are preferable, an acetone-water mixed solvent, acetic acid, acetonitrile, and water are more preferable.

When the compound of the present invention can be in the form of a salt, a pharmaceutically acceptable salt is preferable. Examples of such a pharmaceutically acceptable salt include: for a compound having an acidic group such as a carboxyl group, a salt with alkali metal, such as ammonium salt, sodium, and potassium; a salt with alkaline-earth metal such as calcium; a salt with organic amine, such as magnesium salt, aluminum salt, zinc salt, triethylamine, ethanolamine, morpholine, pyrrolidine, piperidine, piperazine, and dicyclohexylamine; and a salt with basic amino acid, such as arginine, and lysine. For a compound having a basic group, examples of the same include: a salt with inorganic acid, such as hydrochloric acid, sulfuric acid, phosphoric acid, nitric acid, and hydrobromic acid; a salt with organic carboxylic acid, such as acetic acid, citric acid, benzoic acid, maleic acid, fumaric acid, tartaric acid, succinic acid, trifluoroacetic acid, tannic acid, butyric acid, hibenzic acid, pamoic acid, enanthic acid, decanoic acid, teoclic acid, salicylic acid, lactic acid, oxalic acid, mandelic acid, and malic acid; and a salt with organic sulfonic acid, such as methanesulfonic acid, benzenesulfonic acid, and p-toluenesulfonic acid.

In the method of the present invention, a novel compound useful as an intermediate is provided. The compound may be in the form of a salt. Examples of the salt include, in addition to the above examples of pharmaceutically acceptable salts, chemically acceptable salts that are preferable in terms of manufacturing methodes. Examples of the salt include a salt with chemically acceptable acid and a salt with chemically acceptable base.

Examples of a salt with chemically acceptable acid include salts with inorganic acids (e.g., hydrochloric acid, sulfuric acid, phosphoric acid, nitric acid, and hydrobromic acid), organic carboxylic acids (e.g., carbonic acid, acetic acid, citric acid, benzoic acid, maleic acid, fumaric acid, tartaric acid, succinic acid, trifluoroacetic acid, tannic acid, butyric acid, decanoic acid, salicylic acid, lactic acid, oxalic acid, mandelic acid, and malic acid), and organic sulfonic acids (e.g., methanesulfonic acid, p-toluenesulfonic acid, and benzenesulfonic acid).

Examples of a salt with chemically acceptable base include alkali metal salts (e.g., sodium salt, potassium salt, and lithium salt), alkaline-earth metal salts (e.g., calcium salt, and barium salt), and metal salts (e.g., magnesium salt, and aluminum salt).

## EXAMPLES

Hereinafter, the present invention will be described more specifically with reference to Examples. However, the present invention is not intended to be limited to them.

(Analysis conditions)

Each analytical means and analytical apparatuses are as follows.

(1) $^{1}H$ and $^{13}C$ NMR measurements were carried out using TMS as an internal reference material and Avance 400 MHz nuclear magnetic resonance apparatus of Bruker Corporation. Commercially available products of $CDCl_3$, DMSO-$d_6$, and heavy water were directly used.

(2) As a HPLC analyzer, a system composed of the following items was mainly used.

Pump: LC-10AT and LC-10ATvp produced by Shimadzu Corporation
Auto sampler: KMT-100X produced by Kyowa Seimitsu Co., Ltd.
Column oven: AO-30C produced by Shodex and C0631 produced by GL Sciences Inc., U-620 produced by Sugai Chemical Industry Co., Ltd.
UV detector: SPD-10A and SPD-10Avp produced by Shimadzu Corporation HPLC controller: SCL-10A and SCL-10Avp produced by Shimadzu Corporation

(3) HPLC data analysis and processing devices used herein were EZ ChromElite produced by GL Sciences, Inc., (Ver. 2.8.3, Build 2249) and Class-VP (Version 6.10) produced by Shimadzu Corporation and CR-7Aplus produced by Shimadzu Corporation.

(4) For ion chromatography (Cl anion), an apparatus produced by Dionex Corporation (U.S.) (DIONEX, DX-120) was used, and as a data analysis device, CR-7Aplus produced by Shimadzu Corporation was used.

As an eluent for ion chromatography analysis, 1 M $Na_2CO_3$/1 M $NaHCOO_3$=9/1 was used. A sample for analysis was dissolved in ion-free water and then analyzed. As a reference standard for chloride ion ($Cl^-$), potassium chloride was used.

(5) For LC/MS spectrum, UPLC/SQD system and MS detector: SQD were used.
(6) For high resolution mass spectrometry (HRMS), MS700V produced by JEOL Ltd., was used and as FAB matrix, YOKUEDL-FAB-Matrix (m-NBA/DTT mixture) was used.
(7) In the Examples, all raw materials and reagents used herein were commercially available products, and were directly used particularly without purification. 3-amino-N-(tert-butoxycarbonyl)-L-alanine tert-butyl ester hydrochloride (hereinafter, denoted as Boc-DAP-OtBu·HCl) (Watanabe Chemical Industries Ltd.) 3-amino-N-(tert-butoxycarbonyl)-L-alanine methyl ester hydrochloride (hereinafter, denoted as Boc-DAP-OMe·HCl) (Watanabe Chemical Industries Ltd.) 3-amino-N-(benzyloxycarbonyl)-L-alanine methyl ester hydrochloride (hereinafter, denoted as Cbz-DAP-OMe·HCl) (produced by Watanabe Chemical Industries Ltd.). 3-amino-5-chloro-2-hydroxybenzenesulfonic acid (hereinafter, denoted as ACHB) (produced by Tokyo Chemical Industry Co., Ltd.)
3-aminobenzenesulfonic acid (hereinafter, denoted as ABS) (Tokyo Chemical Industry Co., Ltd.)
3-amino-5-chloro-4-methylbenzenesulfonic acid (hereinafter, denoted as ACTS) (produced by Tokyo Chemical Industry Co., Ltd.) N,N'-carbonyldiimidazole (hereinafter, denoted as CDI) (produced by Hodogaya Chemical Co., Ltd.)
Phenyl chloroformate (produced by Tokyo Chemical Industry Co., Ltd.)
(8) HPLC analysis conditions are as follows.

Eluent composition: solution A-0.1% aqueous trifluoroacetic acid solution, solution B-0.1% trifluoroacetic acid-containing acetonitrile, Flow rate: 1.0 mL/min
Detector: UV (254 nm)
Column used: reverse-phase ODS-silica gel column (produced by Shiseido Co., Ltd., CAPCELL PAC type MGII, Column size: inner diameter ϕ 4.6 mm x 150 mm in length, 5 μm, or XR-ODS produced by Shimadzu Corporation, Column size: inner diameter ϕ 3.0 mm x 75 mm in length, 2.2 μm)
Column temperature: 40°C
Gradient analysis conditions:

(solution A/ solution B)= initial (99/1) to 25 minutes later (10/90) to 30 minutes later (10/90),

or

(solution A/ solution B)= initial (99/1) to 12 minutes later (10/90)

Amount of sample injected: 10 μl

In addition, abbreviations used in the Description represent the following meanings.

AcOEt: ethyl acetate
AcOH: acetic acid
ABS: 3-aminobenzenesulfonic acid
ACHB: 3-amino-5-chloro-2-hydroxybenzenesulfonic acid
ACTS: 3-amino-5-chloro-4-methylbenzenesulfonic acid
DAP: 2,3-diaminopropionic acid
IPA: isopropyl alcohol
MeCN: acetonitrile
MsOH: methanesulfonic acid
Py: pyridine
TEA: triethylamine
THF: tetrahydrofuran

(Example 1)

Synthesis of (2S)-2-amino-3-{[(5-chloro-2-hydroxy-3-sulphophenyl)carbamoyl]amino}propanoic acid (compound 1)

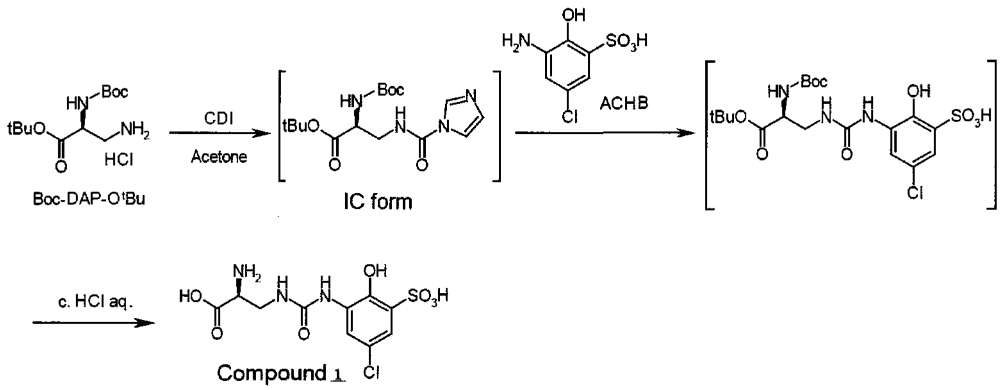


To 150.2 g of CDI (926.6 mmol, 1.1 eq. vs Boc-DAP-O$^t$Bu), 750 mL of acetone (3.0 L/kg) was added and then stirred at 5°C. Boc-DAP-OtBu (250 g) (842.6 mmol) was added in 2 portions, and then 125 mL of acetone (0.5 L/kg) was added to wash. After 30 minutes of stirring, the completion of imidazoyl carbonylation was confirmed by HPLC. ACHB (282.6 g) (1263.8 mmol, 1.5 eq.) was added in 3 portions, and then 125 mL of acetone (0.5 L/kg) was added to wash. The temperature was increased to 30°C, the reaction solution was stirred for 18 hours, and then the completion of the urea forming reaction was confirmed by HPLC. The reaction solution was cooled to 5°C, and then 124.5 mL of concentrated hydrochloric acid (1432.4 mmol, 1.7 eq.) was added, followed by 1 hour of stirring. The precipitated undesired substances were filtered off, and then the precipitated undesired substancese was washed with 1000 mL of acetone (4.0 L/kg). The filtrate was concentrated to an amount of 1018 g (4.1 kg/kg), the temperature was increased to 50°C, and then 625.0 mL of concentrated hydrochloric acid (7187 mmol, 8.5 eq.) was added dropwise. After 30 minutes of stirring, the completion of deprotection was confirmed by HPLC, and then 750 mL of water was added (3.0 L/kg). The solution was concentrated to an amount of 1730g (6.9 kg/kg) under reduced pressure, to precipitate a solid. After 14 hours of stirring at 20°C, filtration under reduced pressure was performed. The filtered solid was washed with 500 mL of acetone (2.0 L/kg), and then dried under reduced pressure at 60°C for 6 hours, to obtain 201.4g of the target product (64.5%).
$^1$H-NMR (400 MHz, DMSO-d6): $\delta$ 8.3 (s, 1H), 8.2 (bs, 3H), 8.1 (d, 1H, J = 2.6 Hz), 7.3 (t, 1H, J = 6.0 Hz), 7.0 (d, 1H, J = 2.6 Hz), 4.0-4.1 (m, 1H), 3.6-3.7 (m, 1H), 3.4-3.5 (m, 1H)

(Example 2)

Synthesis of (2S)-2-amino-3-{[(3-sulphophenyl)carbamoyl]amino}propanoic acid (compound 2)

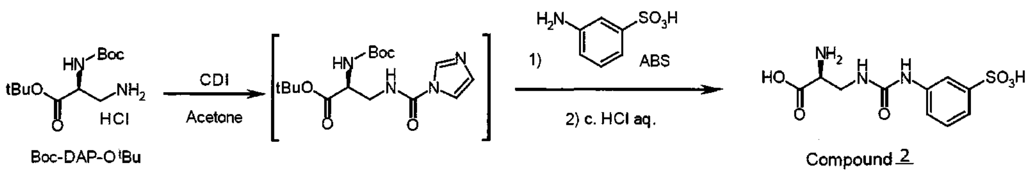


To 120.2 g of CDI (741.2 mmol, 1.1 eq. vs Boc-DAP-OtBu), 600 mL of acetone (3.0 L/kg) was added, and then stirred at 5°C. Boc-DAP-OtBu (200 g) (673.9 mmol) was added in 2 portions, and then 100 mL of acetone (0.5 L/kg) was added to wash. After 30 minutes of stirring, the completion of the imidazoyl carbonylation was confirmed by HPLC. ABS (175.0 g) (1010.8 mmol, 1.5 eq.) was added in 3 portions, and then 100 mL of acetone (0.5 L/kg) was added to wash. The temperature was increased to 30°C, the reaction solution was stirred for 18 hours, and then the completion of the urea forming reaction was confirmed by HPLC. The reaction solution was cooled to 5°C, 99.6 mL of concentrated hydrochloric acid (1145.4 mmol, 1.7 eq.) was added, followed by 1 hour of stirring. The precipitated undesired substances

were filtered off, and then the precipitated undesired substances were washed with 1400 mL of acetone (7.0 L/kg). The filtrate was concentrated to an amount of 800.1 g (4.0 kg/kg), the temperature was increased to 50°C, and then 500.0 mL of concentrated hydrochloric acid (5750.0 mmol, 8.5 eq.) was added dropwise. After 30 minutes of stirring, the completion of deprotection was confirmed by HPLC, and then 600 mL of water (3.0 L/kg) was added. The solution was concentrated to an amount of 1653.7 g under reduced pressure, to precipitate a solid. The solid was aged at 20°C for 15 hours, and then filtered under reduced pressure. The filtered solid was washed with 400 mL of acetone (2.0 L/kg), and then dried under reduced pressure at room temperature for 6 hours, to obtain 140.3 g of the target product (net 132.2 g, 64.7%).
$^1$H-NMR (400 MHz, DMSO-d6): δ 8.8 (s, 1H), 8.2 (bs, 3H), 7.7 (s, 1H), 7.3-7.4 (m, 1H), 7.1-7.2 (m, 2H), 6.3-6.4 (bs, 1H), 4.0-4.1 (bs, 1H), 3.6-3.7 (bs, 1H), 3.5-3.6 (bs, 1H)

(Example 3)

Synthesis of (2S)-2-amino-3-{[(3-chloro-2-methyl-5-sulphophenyl)carbamoyl]amino}propanoic acid (compound 3)

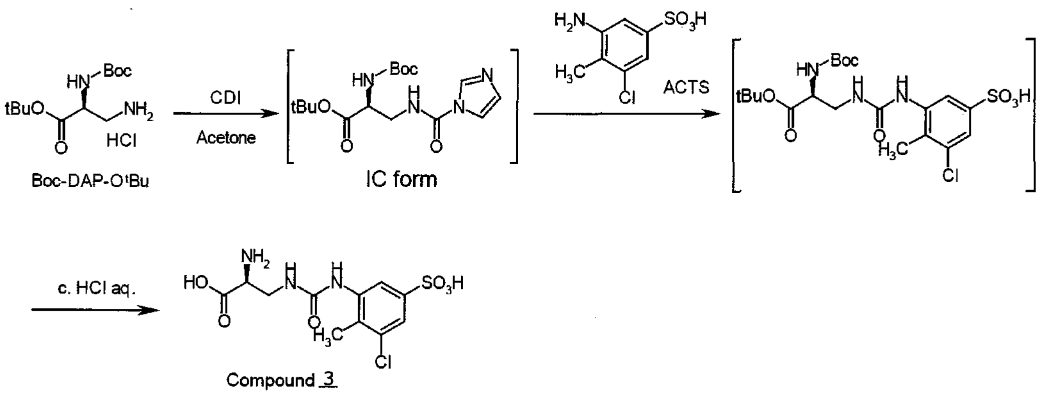


Compound 3

To 14.4 g of CDI (88.8 mmol, 1.05 eq. vs Boc-DAP-OtBu), 75 mL of acetone (3.0 L/kg vs DAP-OtBu) was added and then stirred at 5°C. Boc-DAP-OtBu (25 g) (84.3 mmol) was added in 2 portions. After 30 minutes of stirring, the completion of the imidazoyl carbonylation was confirmed by HPLC. ACTS (26.1 g) (118.0 mmol, 1.4 eq.) was added in 3 portions, and then 25 mL of acetone (1.0 L/kg) was added to wash. The temperature was increased to 30°C, the reaction solution was stirred overnight, and then the completion of the urea forming reaction was confirmed by HPLC. The reaction solution was concentrated at 10 kPa and 40°C to drive off the solvent under reduced pressure, 37.5 mL of water (1.5 L/kg) and 22.8 mL of concentrated hydrochloric acid (257.6 mmol) were added, followed by 2 hours of deprotection. The completion of the reaction was confirmed by HPLC, the reaction solution was cooled to 5°C, and then 60 mL of MeCN (2.4 L/kg) was added, followed by overnight stirring. When 120 mL of MeCN (4.8 L/kg) was further added, separate layers were formed. Hence, 10 mL of water (0.4 L/kg) and 2.5 mL of MeCN (0.1 L/kg) were added. The precipitated solid was filtered under reduced pressure, washed with 60 mL of MeCN/water (1/2), and then dried under reduced pressure at 60°C for 14 hours, to obtain 20.1 g of the target product as a white solid (net 18.3 g, yield 61.8%).
$^1$H-NMR (400 MHz, DMSO-d6): δ 14.70-13.30 (bs, 1H), 8.27 (bs, 3H), 8.15 (s, 1H), 7.98 (d, 1H, J = 1.6 Hz), 7.27 (d, 1H, J = 1.6 Hz), 6.82 (t, 1H, J = 6.0 Hz), 4.04 (bs, 1H), 3.70-3.60 (m, 1H), 3.60-3.50 (m, 1H), 2.22 (s, 3H)

(Example 4)

Synthesis of compound 3 using phenyl chloroformate as carbonyl group-introducing reagent

(Step 1)

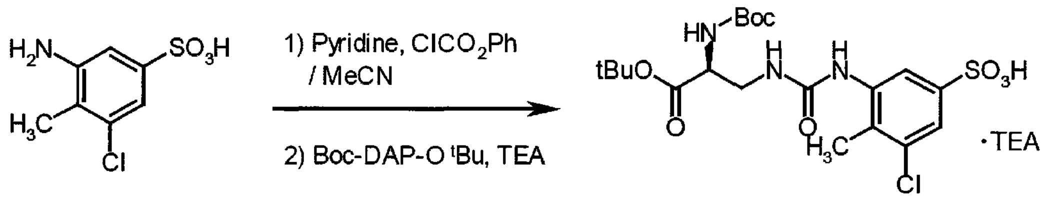


ACTS

Compound 4

To 50 g of ACTS (225.6 mmol), 375 mL of MeCN (7.5 L/kg vs ACTS) and 38.1 mL of Py (473.7 mmol, 2.1 eq.) were added, and then stirred at 25°C. $ClCO_2Ph$ (phenyl chloroformate) (29.9 mL) (236.8 mmol, 1.05 eq.) was added dropwise. After 30 minutes of stirring, the completion of the carbamate forming reaction was confirmed by HPLC. Boc-DAP-OtBu (68.9 g) (232.4 mmol) was added, and then 97.5 mL of TEA (699.3 mmol, 3.1 eq.) was added dropwise. After 3 hours of stirring at 25°C, the completion of the urea forming reaction was confirmed by HPLC. Here, from among the total amount of 517.43 g, 103.5 g of the reaction solution was used and transferred to the next step (down to ACTS 10 g scale).

Water (30 mL) was added and then the solution was concentrated to an amount of 77.0 g at 40°C and 5 kPa. AcOEt (100 mL) (10 L/kg) was added for liquid separation operation, and then 30 mL of water was added to the organic layer to perform liquid separation operation again. The organic layer was concentrated to an amount of 47.6 g at 40°C and 10 kPa, and then 15 mL of AcOEt (1.5 L/kg) and 100 mL of THF (10L/kg) were added. The solution was concentrated again to an amount of 50.7 g, and then THF was added to give the total amount of the solution is 146 g. The solution was concentrated again to an amount of 35.5 g, and then up to 30 mL of AcOEt (3 L/kg) and 100 mL of THF (10 L/kg) were added, to precipitate a solid. The solid was cooled to 5°C, and then aged overnight. The precipitated solid was filtered under reduced pressure, washed with 20 mL of THF (2.0 L/kg), dried at 30°C overnight and then at 40°C for 3 hours under reduced pressure, to obtain 24.9 g of the target product as a white solid (net 23.0 g, 83.6%). $^1$H-NMR (400 MHz, DMSO-d6): $\delta$ 8.86 (bs, 1H), 8.09 (s, 1H), 7.88 (s, 1H), 7.25 (d, 1H, J = 1.6 Hz), 7.14 (d, 1H, J = 7.6 Hz), 6.60 (t, 1H, J = 5.6 Hz), 4.00-3.90 (m, 1H), 3.60-3.50 (m, 1H), 3.30-3.20 (m, 1H), 3.15-3.05 (m, 6H), 2.19 (s, 3H), 1.50-1.30 (m, 18H), 1.20-1.10 (m, 9H)

(Step 2)

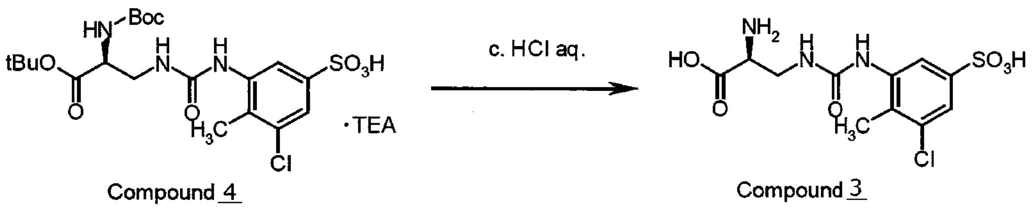


Compound 4

Compound 3

To 21.64 g of compound 4 (net. 20.0g, 32.8 mmol), 68 mL of water (3.4 L/kg vs compound 4) was added. After stirring at 50°C, 12 mL of concentrated hydrochloric acid (135.6 mmol, 4.1 eq.) was added dropwise. After 1 hour of stirring, the temperature was increased to 70°C, and the precipitated solid was dissolved. The completion of the reaction was confirmed by HPLC, the reaction solution was cooled to 50°C, aged for 1 hour, and then cooled to 5°C over 4 hours. The precipitated solid was filtered under reduced pressure, washed with 40 mL of MeCN/water (2/1) (2.0 L/kg), and then dried under reduced pressure at 60°C for 3 hours, to obtain 11.2 g of the target product as a white solid (net 10.5 g, 91.1%).

(Example 5)

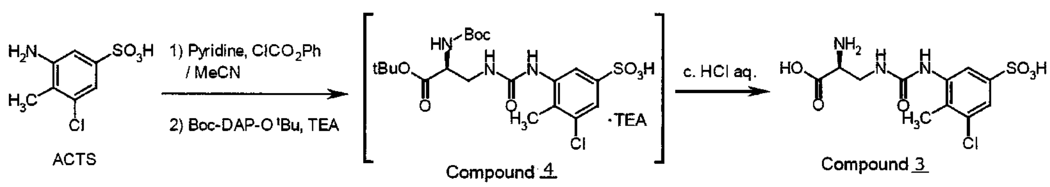


ACTS

Compound 4

Compound 3

To 1.00 g of ACTS (4.51 mmol), 10.0 mL of MeCN (10.0 L/kg vs ACTS) and 0.75 mL of Py (9.25 mmol, 2.05 eq.) were added, and then stirred at 8°C. $ClCO_2Ph$ (0.59 mL) (4.74 mmol, 1.05 eq.) was added dropwise, and then the temperature was increased to room temperature. After 1 hour of stirring, the completion of the carbamate forming reaction was confirmed by HPLC. Boc-DAP-OtBu (1.33 g) (4.51 mmol, 1.0 eq.) was added, and then 1.92 mL of TEA (13.76 mmol, 3.05 eq.) was added dropwise, followed by 1 hour of stirring at 40°C. The completion of the urea forming reaction was confirmed by HPLC, and then the reaction solution was concentrated to drive off the solvent. Water (1.0 mL) and 2.0 mL of concentrated hydrochloric acid (22.6 mmol, 5.0 eq.) were added and then the mixture was stirred at 50°C for 4 hours. The completion of deprotection was confirmed by HPLC, 7.5 mL of MeCN (7.5 L/kg), and 4.5 mL of 1M HCl aq. were added, followed by overnight stirring at 5°C. The precipitated solid was filtered under reduced pressure, washed with 3.0 mL of MeCN (3.0 L/kg), and then dried at 60°C overnight, to obtain 1.28 g of the target product as a white solid (net 1.18 g, 77.0%).

(Example 6)

(Step 1)

Synthesis of 3-({[(2S)-2-amino-3-methoxy-3-oxopropyl]carbamoyl}amino)-5-chloro-4-methylbenzene-1-sulfonic acid (compound 5)

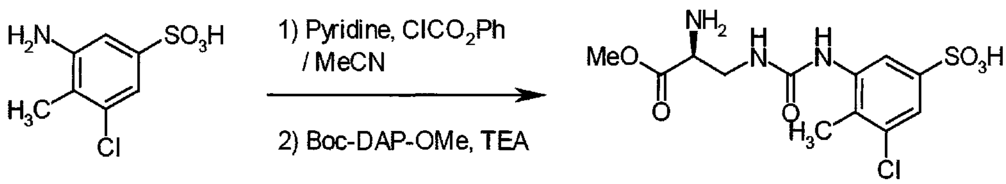


ACTS

Compound 5

To 5 g of ACTS (22.56 mmol), 37.5 mL of MeCN (7.5 L/kg vs ACTS) and 3.81 mL of Py (47.38 mmol, 2.1 eq.) were added, and then stirred at 25°C. $ClCO_2Ph$ (2.99 mL) (23.68 mmol, 1.05 eq.) was added dropwise. After 30 minutes of stirring, the completion of the carbamate forming reaction was confirmed by HPLC. Boc-DAP-OMe (5.92 g) (23.23 mmol, 1.03 eq.) was added, and then 9.75 mL of TEA (69.93 mmol, 3.1 eq.) was added dropwise, followed by 3 hours of stirring at 25°C. Boc-DAP-OMe (0.4 g) (1.58 mmol, 0.07 eq.), 0.22 mL of TEA (1.58 mmol, 0.07 eq.) were further added, and then the completion of the urea forming reaction was confirmed by HPLC. MsOH (7.32 mL) (112.8 mmol, 5.0 eq.) was added, and then the temperature was increased to 50°C, followed by 4 hours of stirring. The completion of deprotection was confirmed by HPLC, the reaction solution was cooled to 25°C, 37.5 mL of MeCN (7.5 L/kg) and 7.5 mL of water (1.5 L/kg) were added to precipitate a solid. The solid was cooled to 5°C and then aged for 16 hours. The precipitated solid was filtered under reduced pressure, washed with 20 mL of water/MeCN (1/2) (4.0 L/kg), and then dried under reduced pressure at 40°C for 5 hours, to obtain 7.72 g of the target product as a white solid (net 7.20 g, 87.3%).
$^1H$-NMR (400 MHz, DMSO-d6): $\delta$ 8.39 (bs, 3H), 8.16 (d, 1H, J = 1.2 Hz), 7.90 (d, 1H, J = 1.6 Hz), 7.28 (d, 1H, J = 1.6 Hz), 6.78 (t, 1H, J = 5.6 Hz), 4.20-4.10 (m, 1H), 3.77 (s, 3H), 3.70-3.60 (m, 1H), 3.55-3.45 (m, 1H), 2.21 (s, 3H)
HRMS ($FAB^-$): calcd for m/z 364.0369 (M-H), found m/z 364.0395 (M-H)

(Step 2)

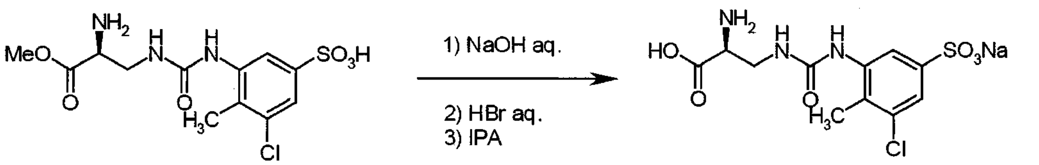


Compound 5

Compoundl 3 (Na salt)

To 10.64 g of compound 5 (net 10.0 g, 27.34 mmol), 18 mL of water (1.8 L/kg vs compound 5) was added and

then stirred at 8°C. A 48% aqueous sodium hydroxide solution (3.42 mL) (57.41 mmol, 2.1 eq.) was added dropwise, and then 1.0 mL of water (1.0 L/kg) was added to wash. After 15 minutes of stirring at 8°C, the completion of hydrolysis was confirmed by HPLC, the temperature was increased to 25°C, and then about 3.55 mL of 48% HBr aq. was added to adjust the pH to 5.8. IPA (65 mL) (6.5 L/kg) was added dropwise, the precipitation of the target product was confirmed, and then the product was aged for 1 hour. IPA (81 mL) (8.1 L/kg) was added dropwise and then the product was aged overnight at 8°C. The precipitated solid was filtered under reduced pressure, washed with 20 mL of IPA (2.0 L/kg), and then dried under reduced pressure at 40°C for 4 hours, to obtain 10.7 g of the target product as a white solid (net 9.46 g, 92.6%).
$^1$H-NMR (400 MHz, DMSO-d6): δ8.76 (s, 1H), 7.91 (d, 1H, J = 1.6 Hz), 8.00-7.50 (bs, 2H), 7.24 (d, 1H, J = 1.6 Hz), 7.20 (t, 1H, J = 5.6 Hz), 3.58-3.54 (m, 1H), 3.47-3.43 (m, 1H), 3.42-3.37 (m, 1H), 2.23 (s, 3H)

(Example 7)

(Step 1)

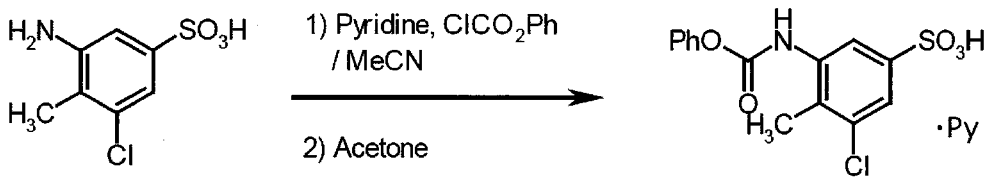


ACTS

Compound 6

To 10.0 g of ACTS (45.1 mmol), 50 mL of MeCN (5.0 L/kg vs ACTS) and 7.46 mL of Py (92.5 mmol, 2.05 eq.) were added and then stirred at 8°C. $ClCO_2Ph$ (5.98 mL) (47.4 mmol, 1.05 eq.) was added dropwise, and then the temperature was increased to 25°C. After 1 hour of stirring, the completion of the carbamate forming reaction was confirmed by HPLC. Acetone (100 ml) (10.0 L/kg vs ACTS) was added, the reaction solution was cooled to 8°C and then aged for 1 hour. The precipitated solid was filtered under reduced pressure, washed with 30 mL of acetone (3.0 L/kg vs ACTS), and then dried under reduced pressure at 60°C for 2 hours, to obtain 17.8 g of the target product (in a free form, net 14.4 g, quant).
$^1$H-NMR (400 MHz, DMSO-d6): δ 9.76 (bs, 1H), 8.93-8.90 (m, 2H), 8.60-8.50 (m, 1H), 8.10-8.00 (m, 2H), 7.60 (s, 1H), 7.50-7.40 (m, 3H), 7.30-7.20 (m, 3H), 2.30 (s, 3H)

(Step 2)

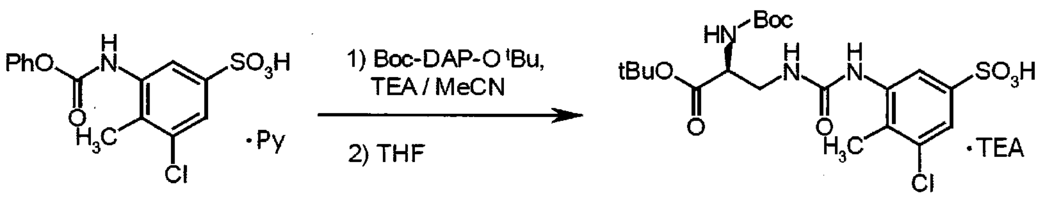


Compound 6

Compound 4

To 5.0 g of compound 6 (11.9 mmol), 50 ml of acetonitrile and 3.53 g of Boc-DAP-OtBu (11.9 mmol) were added, and then stirred at 8°C. Triethylamine (3.5 ml) (25 mmol) was added dropwise, and then the mixture was stirred overnight at room temperature. Under reduced pressure, the solvent was evaporated, and then 25 ml of ethyl acetate and 5 ml of water were added for extraction. The organic layer was washed with 5 ml of water, the solvent was evaporated, and then 50 ml of tetrahydrofuran was added. The solution was cooled to 8°C, and then aged for 1 hour. The precipitated solid was filtered under reduced pressure, washed with 10 ml of tetrahydrofuran, and then dried overnight at 60°C under reduced pressure, to obtain 6.3 g of the target product as a white solid.

(Example 8)

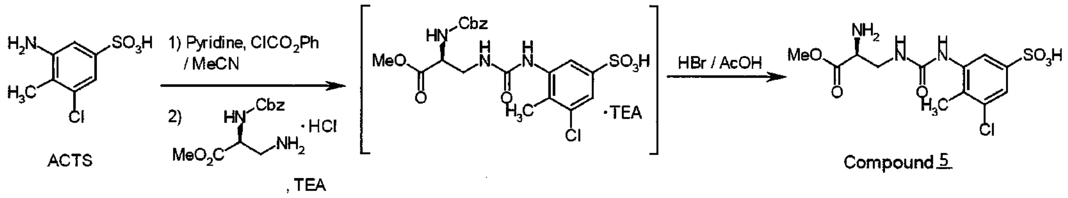


ACTS

Compound 5

To 1.08 g of ACTS (4.89 mmol), 8.1 mL of MeCN (7.5 L/kg vs ACTS) and 827 μL of Py (10.27 mmol, 2.1 eq.) were added and then stirred at room temperature. $ClCO_2Ph$ (649 μL) (5.14 mmol, 1.05 eq.) was added dropwise. After 30 minutes of stirring, the completion of the carbamate forming reaction was confirmed by HPLC. Cbz-DAP-OMe·HCl (1.48 g) (5.04 mmol, 1.03 eq.) was added, and then 2.1 mL of TEA (15.17 mmol, 3.1 eq.) was added dropwise. After about 5 hours of stirring at room temperature, the completion of the urea forming reaction was confirmed by HPLC, the reaction solution was concentrated to drive off the solvent, and then 15.0 mL of 30% HBr/AcOH was added. After 70 minutes of stirring at room temperature, the completion of deprotection was confirmed by HPLC. After concentration to dryness, 10 mL of water and 4 mL of AcOEt were added for extraction operation. An aqueous layer was stirred overnight at room temperature. The precipitated solid was filtered under reduced pressure, washed with 15 mL of water, and 10 mL of AcOEt, and then dried at 40°C for 3 hours, to obtain 1.45 g of the target product as a white solid (58.8%).

(Example 9)

Synthesis of compound 7 using phenyl chloroformate as carbonyl group-introducing reagent (compound 1 in the form of methyl ester)

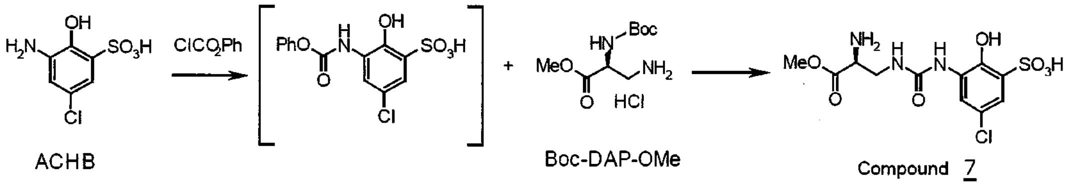


ACHB

Boc-DAP-OMe

Compound 7

To 5.00 g of ACHB (22.4 mmol), 73 mL of MeCN (14.6 L/kg vs ACHB) and 3.8 mL of Py (47 mmol, 2.1 eq.) were added and then stirred at 40°C. $ClCO_2Ph$ (3.0 mL) (24 mmol, 1.05 eq.) was added dropwise. After 30 minutes of stirring, the completion of the carbamate forming reaction was confirmed by HPLC. Boc-DAP-OMe (5.87 g) (23 mmol, 1.0 eq.) was added washing with a small amount of MeCN. TEA (9.7 mL) (70 mmol, 3.1 eq.) was added dropwise, and then stirred at 40°C for 3 hours. The completion of the urea forming reaction was confirmed by HPLC, and then the reaction solution was cooled to room temperature. MsOH (7.3 mL) (112 mmol, 5.0 eq.) was added, and then the temperature was increased to 50°C, followed by 7 hours of stirring. Furthermore, 1.5 mL of MsOH (23 mmol, 1.0 eq.) was added for overnight reaction at 50°C. The completion of deprotection was confirmed by HPLC, 90 mL of acetone was added to the reaction solution, and then the solution was cooled to room temperature. The precipitated solid was obtained and then dried under reduced pressure at 60°C, to obtain the target product. $^1$H-NMR (400 MHz, DMSO-d6): δ 7.22 (m, 1H), 7.14 (m, 1H), 4.36 (m, 1H), 3.80 (s, 3H), 3.20-3.40 (m, 2H).

(Example 10)

Synthesis of compound 5 using 4-chlorophenyl chloroformate as carbonyl group-introducing reagent

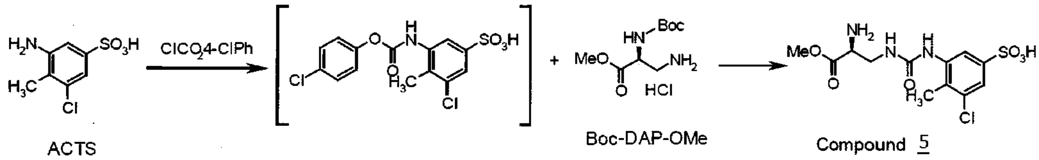


ACTS Boc-DAP-OMe Compound 5

To 5.00 g of ACTS (22.6 mmol), 73 mL of MeCN (14.6 L/kg vs ACTS) and 3.8 mL of Py (47 mmol, 2.1 eq.) were added and then stirred at 40°C. 4-chlorophenyl chloroformate (3.25 mL) (23.7 mmol, 1.05 eq.) was added dropwise. After 1.5 hours of stirring at 40°C, the completion of the carbamate forming reaction was confirmed by HPLC. Boc-DAP-OMe (5.92g) (23.2 mol, 1.0 eq.) was added washing with a small amount of MeCN. TEA (9.7 mL) (70 mmol, 3.1 eq.) was added dropwise, and then stirred at 40°C for 2 hours. The completion of the urea forming reaction was confirmed by HPLC, and then the reaction solution was cooled to room temperature. MsOH (7.3 mL) (113 mmol, 5.0 eq.) was added, and then the temperature was increased to 50°C, followed by 3.5 hours of stirring. The completion of deprotection was confirmed by HPLC, and then the reaction solution was cooled to room temperature. Water (7.5 mL) was added, the solution was cooled to 8°C and then stirred overnight. The precipitated solid was filtered, washed with a small amount of MeCN water, and then dried at 60°C overnight, to obtain 6.94 g of the target product as a white solid (84.1 %).

(Example 11)

Synthesis of compound 5 using 4-nitrophenyl chloroformate as carbonyl group-introducing reagent

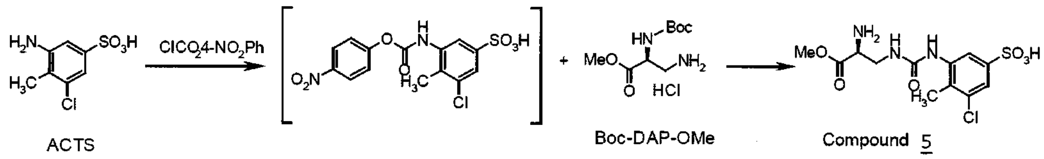


ACTS Boc-DAP-OMe Compound 5

To 5.00 g of ACTS (22.6 mmol), 73 mL of MeCN (14.6 L/kg vs ACTS) and 3.8 mL of Py (47 mmol, 2.1 eq.) were added and then stirred at 40°C. 4-nitrophenyl chloroformate (4.77 mL) (23.7 mmol, 1.05 eq.) was added dropwise. After 3.5 hours of stirring at 40°C, the completion of the carbamate forming reaction was confirmed by HPLC. Boc-DAP-OMe (5.92 g) (23.2 mmol, 1.0 eq.) was added washing with a small amount of MeCN, and then 9.7 mL of TEA (70 mmol, 3.1 eq.) was added dropwise. After 2 hours of stirring at 40°C, the completion of the urea forming reaction was confirmed by HPLC, and then the reaction solution was cooled to room temperature. MsOH (7.3 mL) (113 mmol, 5.0 eq.) was added, and then the temperature was increased to 50°C, followed by 3.5 hours of stirring. The completion of deprotection was confirmed by HPLC, the reaction solution was cooled to room temperature, 7.5 mL of water was added, the reaction solution was cooled to 8°C and then stirred overnight. The precipitated solid was filtered, washed with a small amount of MeCN water, and then dried overnight at 60°C, to obtain 5.96 g of the target product as a white solid (72.2%).

(Example 12)

Synthesis of compound 3 using Boc-DAP-OH

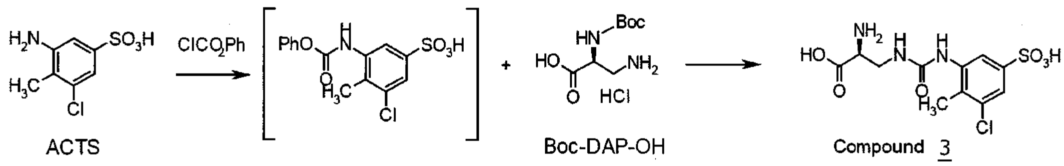


ACTS Boc-DAP-OH Compound 3

To 5.00 g of ACTS (22.6 mmol), 73 mL of MeCN (14.6 L/kg vs ACTS) and 3.8 mL of Py (47 mmol, 2.1 eq.) were added and then stirred at 40°C. Phenyl chloroformate (3.00 mL) (23.8 mmol, 1.05 eq.) was added dropwise. After 0.5 hours of stirring at 40°C, the completion of the carbamate forming reaction was confirmed by HPLC (carbamate

forming reaction product: 4.37 minutes, ACTS: N.D.). Boc-DAP-OH (4.75 g) (23.2 mmol, 1.0 eq.) was added washing with a small amount of MeCN, and then 9.7 mL of TEA (70 mmol, 3.1 eq.) was added dropwise. After 2 hours of stirring at 40°C, the completion of the urea forming reaction was confirmed by HPLC (urea forming reaction product: 3.81 minutes, carbamate forming reaction product: 0.02 area% vs urea forming reaction product), and then the reaction solution was cooled to room temperature. MsOH (7.3 mL) (113 mmol, 5.0 eq.) was added, and then the temperature was increased to 50°C, followed by 4.5 hours of stirring. Moreover, 1.5 mL of MsOH (23 mmol, 1.0 eq.) was added and then stirred for 1 hour, and thus the generation of the target product was confirmed by HPLC (compound 3: 2.49 minutes, urea forming reaction product: 0.50 area% vs compound 3, the area of compound 3 with respect to the total area excluding pyridine: 71.0 area%).

## Claims

1. A method for producing a compound represented by formula (I) or a salt thereof:

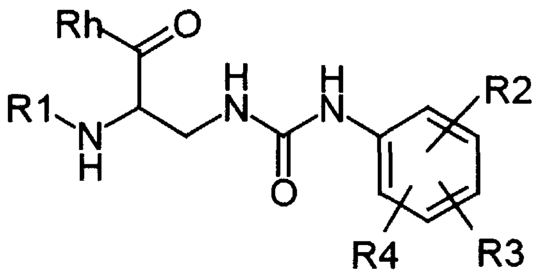

(I)

wherein R1 represents a hydrogen atom,
Rh represents a hydroxy group, a $C_{1-6}$ alkoxy group, or a benzyloxy group,
R2 represents a sulfo group,
R3 and R4 each independently represent a hydrogen atom, a substituted or unsubstituted $C_{1-6}$ alkyl group, a halogen atom, a hydroxy group, a $C_{1-6}$ alkoxy group, a nitro group, or an amino group,
comprising the following steps (a) and (b):

(a) dissolving or suspending a compound represented by formula (II) or a salt thereof:

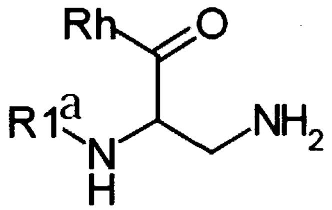

(II)

wherein $R1^a$ represents a hydrogen atom, or a protecting group for an amino group, a carbonyl group-introducing reagent, and a compound represented by formula (III) or a salt thereof:

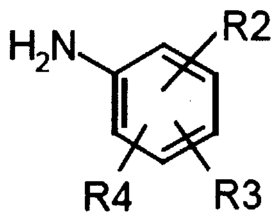

(III)

in a solvent, for reaction in the presence or the absence of a base; and
(b) deprotecting as necesssary the reaction product obtained in step (a).

2. The production method according to claim 1, wherein in formula (II), $R1^a$ represents a benzyloxycarbonyl group or a t-butoxycarbonyl group.

3. The production method according to claim 1 or 2, wherein in formula (III), R3 and R4 each independently represent a hydrogen atom, an unsubstituted $C_{1-6}$ alkyl group, a halogen atom, or a hydroxy group.

4. The production method according to any one of claims 1 to 3, wherein the carbonyl group-introducing reagent is a chloroformic ester, carbonyldiimidazole, phosgene, triphosgene, or dimethyl carbonate.

5. The production method according to any one of claims 1 to 3, wherein in step (a), the carbonyl group-introducing reagent is carbonyldiimidazole, the base is absent, and the solvent is one or two or more solvents selected from acetone, methyl ethyl ketone, methyl isobutyl ketone, dichloromethane, tetrahydrofuran and acetonitrile.

6. The production method according to any one of claim 1 to 3, wherein in step (a), the carbonyl group-introducing reagent is a chloroformic ester, the base is one or two or more bases selected from triethylamine, pyridine, and diisopropylethylamine, and the solvent is one or two or more solvents selected from acetonitrile, propionitrile, dichloromethane, acetone, N,N-dimethylformamide and tetrahydrofuran.

7. The production method according to claim 6, wherein the chloroformic ester is methyl chloroformate, ethyl chloroformate, phenyl chloroformate, 4-chlorophenyl chloroformate or 4-nitrophenyl chloroformate.

8. A method for producing a compound represented by formula (I), or a salt thereof:

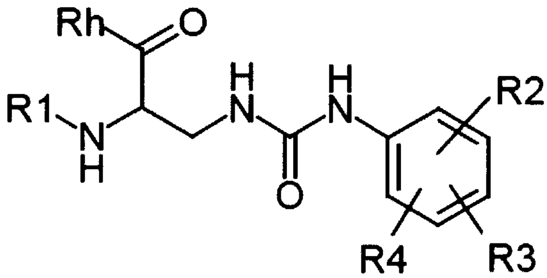

( I )

wherein R1 represents a hydrogen atom,
Rh represents a hydroxy group, a $C_{1-6}$ alkoxy group, or a benzyloxy group,
R2 represents a sulfo group,
R3 and R4 each independently represent a hydrogen atom, a substituted or unsubstituted $C_{1-6}$ alkyl group, a halogen atom, a hydroxy group, a $C_{1-6}$ alkoxy group, a nitro group, or an amino group,
comprising the following steps (a-1), (a-2) and (b):

(a-1) dissolving or suspending a compound represented by formula (III) or a salt thereof:

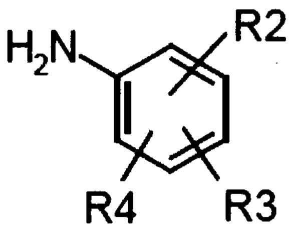

(III)

in a solvent with a chloroformic ester, for reaction in the presence or the absence of a base, to obtain a reaction product containing a compound of formula (IVb) or a salt thereof:

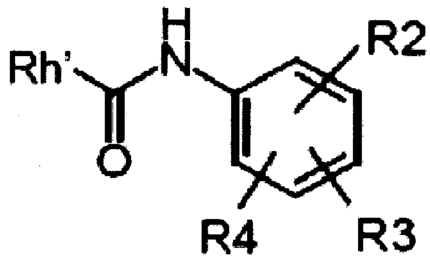 (IVb)

wherein Rh' represents a $C_{1-6}$ alkoxy group, a benzyloxy group, or a phenoxy group,
(a-2) reacting the reaction product obtained in step (a-1) containing the compound of formula (IVb) or a salt thereof, with a compound represented by formula (II) or a salt thereof:

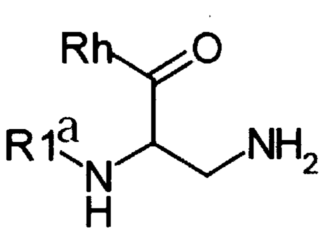 (II)

wherein $R1^a$ represents a hydrogen atom, or a protecting group for an amino group, and
(b) deprotecting as necessary the reaction product obtained in step (a-2).

**9.** The production method according to any one of claims 1 to 8, wherein $R1^a$ represents a tert-butoxycarbonyl group, and in step (b), the deprotecting reagent which is hydrochloric acid, sulfuric acid, p-toluenesulfonic acid, methanesulfonic acid, or trifluoroacetic acid is used.

**10.** The production method according to any one of claims 1 to 8, wherein $R1^a$ represents a benzyloxycarbonyl group, and in step (b), the deprotecting reagent which is hydrogen bromide/acetic acid is used, or a hydrogenation reaction is performed using palladium carbon.

**11.** The production method according to any one of claims 1 to 8, wherein Rh represents a tert-butoxy group, and in step (b), the deprotecting reagent which is hydrochloric acid, formic acid, p-toluenesulfonic acid, trifluoroacetic acid, or potassium hydroxide is used.

**12.** The production method according to any one of claims 1 to 8, wherein Rh represents a methoxy group, and in step (b), the deprotecting reagent which is sodium hydroxide, potassium hydroxide, or lithium hydroxide is used.

**13.** A compound represented by the following formula or a salt thereof:

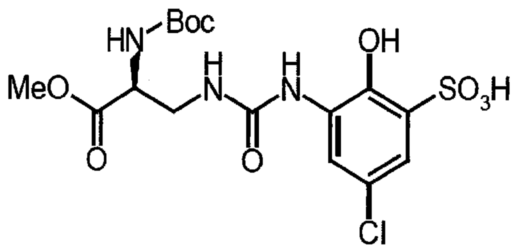

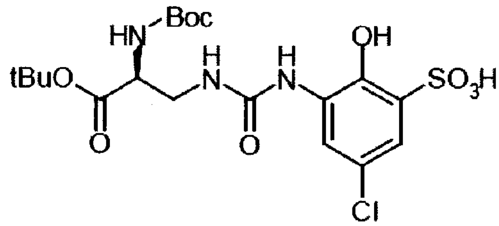

,

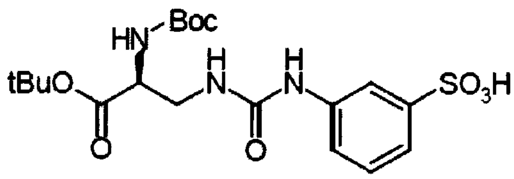

,

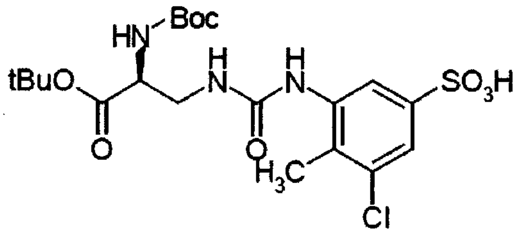

,

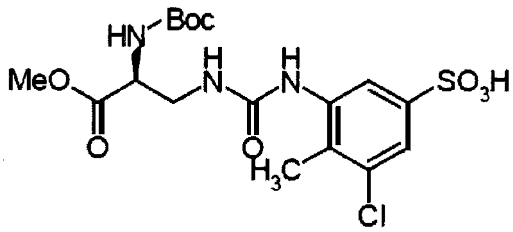

or

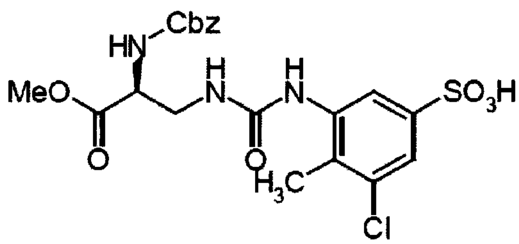

**14.** A compound represented by the following formula or a salt thereof:

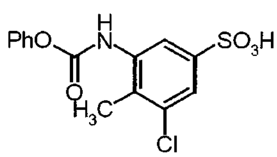

,

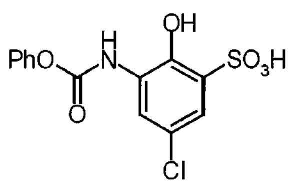

,

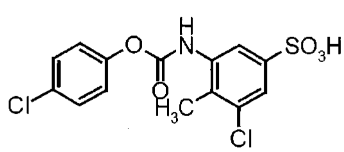

or

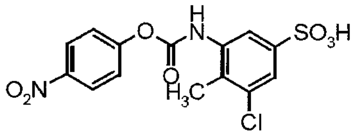

## INTERNATIONAL SEARCH REPORT

International application No. PCT/JP2016/065945

A. CLASSIFICATION OF SUBJECT MATTER
*C07C303/22*(2006.01)i, *C07C309/46*(2006.01)i

According to International Patent Classification (IPC) or to both national classification and IPC

B. FIELDS SEARCHED

Minimum documentation searched (classification system followed by classification symbols)
C07C303/22, C07C309/46

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
CASREACT(STN), CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2013-63971 A (Ajinomoto Co., Inc.), 11 April 2013 (11.04.2013), paragraphs [0037] to [0038], [0068]; preparation examples 13, 77 (Family: none) | 1-5,13<br>6-12,14 |
| Y | SUN,W. et al, 6,6-Fused heterocyclic ureas as highly potent TRPV1 antagonists, Bioorganic & Medicinal Chemistry Letters, 2015.01.05, Vol.25, No.4, p.803-806, ISSN: 0960-894X | 6-8,14 |
| Y | LUO,G. et al, Isosteric N-arylpiperazine replacements in a series of dihydropyridine NPY1 receptor antagonists, Bioorganic & Medicinal Chemistry Letters, 2004, Vol.14, No.24, p.5975-5978, ISSN: 0960-894X | 6-8,14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>18 August 2016 (18.08.16) | Date of mailing of the international search report<br>30 August 2016 (30.08.16) |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

International application No.
PCT/JP2016/065945

C (Continuation). DOCUMENTS CONSIDERED TO BE RELEVANT

| Category* | Citation of document, with indication, where appropriate, of the relevant passages | Relevant to claim No. |
|---|---|---|
| Y | LI,R. et al, Fragment-Based and Structure-Guided Discovery and Optimization of Rho Kinase Inhibitors, Journal of Medicinal Chemistry, 2012, Vol.55, No.5, p.2474-2478, ISSN: 0022-2623 | 6-8,14 |
| Y | 4th edition Jikken Kagaku Koza 22, Yuki Gosei IV, Maruzen Co., Ltd., 1992, pages 271 to 281 | 9-12 |
| A | SHEPPARD,G.S. et al, Discovery and Optimization of Anthranilic Acid Sulfonamides as Inhibitors of Methionine Aminopeptidase-2: A Structural Basis for the Reduction of Albumin Binding, Journal of Medicinal Chemistry, 2006, Vol.49, No.13, p.3832-3849, ISSN: 0022-2623 | 1-14 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011108690 A
- JP 2013063971 A


### Non-patent literature cited in the description


- *Current Opinion Pharmacology,* 2002, vol. 2, 734-739
- REGPARA tablets (R)25mg/REGPARA tablets (R) 75 mg. documents attached to medical drugs. January 2010